(19) Europäisches Patentamt / European Patent Office / Office européen des brevets

(11) EP 4 089 407 A1

(12) **EUROPEAN PATENT APPLICATION**

(43) Date of publication:
**16.11.2022 Bulletin 2022/46**

(21) Application number: **22165000.5**

(22) Date of filing: **29.03.2022**

(51) International Patent Classification (IPC):
**G01N 27/407** (2006.01)    G01N 33/00 (2006.01)

(52) Cooperative Patent Classification (CPC):
**G01N 27/4078;** G01N 33/0037

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.05.2021 JP 2021079639**

(71) Applicant: **NGK SPARK PLUG CO., LTD.**
**Mizuho-ku**
**Nagoya-shi**
**Aichi 467-8525 (JP)**

(72) Inventors:
• **Liu, Sunchao**
  **Nagoya-shi, Aichi, 467-8525 (JP)**
• **Oba, Takehiro**
  **Nagoya-shi, Aichi, 467-8525 (JP)**

(74) Representative: **Diehl & Partner**
**Patent- und Rechtsanwaltskanzlei mbB**
**Erika-Mann-Straße 9**
**80636 München (DE)**

(54) **GAS SENSOR**

(57)     In a gas sensor (1) including a sensor element (5), a metal shell (3), an outer casing (11), metal terminals (41 to 44), a separator (13), lead wires (31 to 34), and a rubber cap (15), the separator (13) is interposed and fixed between the rubber cap (15) located on a rear side with respect to the separator (13) and elastically compressed in an axial direction DX and a front side member (67) located on a front side with respect to the separator (13). A compression set of the rubber cap (15) after the rubber cap (15) is compressed by 25% in the axial direction DX in a temperature environment of 250°C for 67 hours is 32.3% or less.

FIG. 1

EP 4 089 407 A1

**Description**

BACKGROUND

TECHNICAL FIELD

The present invention relates to a gas sensor.

BACKGROUND ART

Conventionally, a gas sensor that is mounted on an exhaust pipe of an automobile or the like when being used and that detects a specific gas component (for example, oxygen, NOx, or the like) in a gas to be measured (exhaust gas), has been known. As such a gas sensor, Patent Literature 1 discloses a gas sensor including: a sensor element which extends in an axial direction and which has an electrode terminal portion on a rear side of the sensor element; a metal shell which surrounds the periphery of the sensor element; and an outer casing which is made of metal, which has a tubular shape extending in the axial direction, and which is mounted on a portion on the rear side of the metal shell.
The gas sensor further includes: a metal terminal which extends in the axial direction and which has a front side connection portion to which the electrode terminal portion of the sensor element is connected; a separator which has a tubular shape extending in the axial direction, which is disposed inside the outer casing so as to be spaced apart from an inner peripheral surface of the outer casing, and in which the front side connection portion of the metal terminal and a portion, of the sensor element, having the electrode terminal portion are housed; a lead wire which is connected to a rear side connection portion, of the metal terminal, located on the rear side of the metal terminal and which extends to the rear side of the gas sensor; and a rubber cap which has a tubular shape extending in the axial direction and which is fixed to the outer casing so as to close a rear side opening of the outer casing. The separator includes: a separator body portion which has a tubular shape and which is obtained by combining two insulating members made of insulating ceramic; and a body portion holding portion (portion which surrounds the periphery of the separator body portion to hold the tubular shape of the separator body portion) which holds a state where the two insulating members are combined into the separator body portion having a tubular shape. In the gas sensor, the rear side connection portion of the metal terminal is inserted in a through hole which penetrates the rubber cap in the axial direction, and a portion, of the lead wire, which is located on the rear side with respect to the rear side connection portion and which is inserted in the through hole of the rubber cap is fixed in close contact with the through hole.

CITATION LIST

PATENT LITERATURE

Patent Literature 1: Japanese Laid-Open Patent Publication No. 2019-2936

SUMMARY

TECHNICAL PROBLEM

Meanwhile, in the above-described gas sensor, the separator is spaced apart from an inner casing which is a front side member. The front side member is a member that is located closest to the separator among members located on the front side with respect to the separator and in which the sensor element is inserted in the axial direction. Furthermore, the separator is also spaced apart from the rubber cap which is a rear side member. The rear side member is a member that is located closest to the separator among members located on the rear side with respect to the separator. Therefore, in the above-described gas sensor, the separator is not fixed inside the outer casing.
Therefore, when a force is applied from the outside to the above-described gas sensor or vibration is transmitted from the outside to the above-described gas sensor, the separator may vibrate relative to the outer casing in a direction (for example, the radial direction of the outer casing) crossing the axial direction. Accordingly, the front side connection portion of the metal terminal which is housed inside the separator may vibrate relative to the outer casing in the direction (referred to as an axial line crossing direction) crossing the axial direction. On the other hand, the rubber cap which is fixed to the outer casing does not vibrate (or is less likely to vibrate) relative to the outer casing, so that the rear side connection portion of the metal terminal which is inserted in the rubber cap also does not vibrate (or is less likely to vibrate) relative to the outer casing.
Therefore, in the metal terminal which extends in the axial direction, the front side connection portion of the metal terminal may vibrate relative to the rear side connection portion in the axial line crossing direction, whereby stress (for example, shear stress) may occur between the front side connection portion and the rear side connection portion, and the metal terminal may be broken between the front side connection portion and the rear side connection portion. Due to this, the gas sensor is required to reduce vibration of the separator relative to the outer casing in the axial line crossing direction (direction crossing the axial direction).

The present invention has been made in view of such circumstances, and an object of the present invention is to provide a gas sensor in which a separator is less likely to vibrate relative to an outer casing in an axial line crossing direction.

SOLUTION TO PROBLEM

An aspect of the present invention is directed to a gas sensor including: a sensor element which extends in an axial direction and which has an electrode terminal portion on a rear side of the sensor element; a metal shell which surrounds a periphery of the sensor element; an outer casing made of metal, which has a tubular shape extending in the axial direction, and which is mounted on a portion on the rear side of the metal shell; a metal terminal which extends in the axial direction and which has a front side connection portion to which the electrode terminal portion of the sensor element is connected; a separator which has a tubular shape extending in the axial direction, which is disposed inside the outer casing so as to be spaced apart from an inner peripheral surface of the outer casing, and in which the front side connection portion of the metal terminal and a portion, of the sensor element, having the electrode terminal portion are housed; a lead wire which is connected to a rear side connection portion, of the metal terminal, located on the rear side of the metal terminal, and which extends to the rear side of the gas sensor; and a rubber cap which is fixed to the outer casing so as to close a rear side opening of the outer casing, characterized in that the separator is interposed and fixed between the rubber cap located on the rear side with respect to the separator and elastically compressed in the axial direction and a front side member located on a front side with respect to the separator, and a compression set of the rubber cap after the rubber cap is compressed by 25% in the axial direction in a temperature environment of 250°C for 67 hours is 32.3% or less.

The above-described gas sensor includes the rubber cap fixed to the outer casing so as to close the rear side opening of the outer casing. Moreover, in the above-described gas sensor, the separator is interposed and fixed between the rubber cap located on the rear side with respect to the separator and elastically compressed in the axial direction and the front side member located on the front side with respect to the separator. That is, the separator is interposed and fixed between the rubber cap and the front side member so as to be pressed to the front side directly or indirectly via another member by the rubber cap elastically compressed in the axial direction. As described above, the separator is interposed and fixed between the rubber cap and the front side member while being pressed to the front side by the elastic reaction force of the rubber cap elastically compressed in the axial direction. Therefore, in the above-described gas sensor, the separator is fixed by the rubber cap fixed to the outer casing. In other words, the separator is fixed to the outer casing via the rubber cap.

Therefore, in the above-described gas sensor, when a force is applied from the outside to the gas sensor or vibration is transmitted from the outside to the gas sensor, "vibration of the separator relative to the outer casing in a direction (for example, the radial direction of the outer casing) crossing the axial direction" is less likely to occur. Therefore, the above-described gas sensor is a gas sensor in which the separator is less likely to vibrate relative to the outer casing in an axial line crossing direction.

Examples of the mode in which the separator is interposed between the rubber cap and the front side member include a mode in which only the separator is interposed between the rubber cap and the front side member as a mode in which the separator is in direct contact with the rubber cap (mode in which the separator is pressed to the front side directly by the rubber cap), and a mode in which the separator and the rubber cap are interposed with another member therebetween and the separator and the other member are interposed between the rubber cap and the front side member as a mode in which the separator is pressed to the front side indirectly by the rubber cap via the other member.

Meanwhile, in the above-described gas sensor, as described above, the separator is fixed by using the elastic reaction force of the rubber cap elastically compressed in the axial direction. Therefore, in the case where a rubber cap having a large compression set in the axial direction is used, the elastic reaction force of the rubber cap may be significantly reduced due to the use of the gas sensor, and it may become impossible to properly fix the separator.

In this regard, in the above-described gas sensor, as the rubber cap, a rubber cap whose compression set (compression set in the axial direction) after the rubber cap is compressed by 25% in the axial direction for 67 hours in a 250°C environment is 32.3% or less, is used. Specifically, the compression set of the rubber cap measured under the measurement conditions of a measurement temperature of 250°C, a measurement time of 67 hours, and a compression rate of 25% in accordance with JIS K6262 is 32.3% or less. Accordingly, a reduction in the elastic reaction force of the rubber cap due to the use of the gas sensor (in particular, use in a high temperature environment) can be decreased, so that a state where the separator is fixed can be maintained over a long period of time.

The compression set of the rubber cap can be measured under the measurement conditions of a measurement temperature of 250°C, a measurement time of 67 hours, and a compression rate of 25% in accordance with JIS K6262 by a measurement method described in (1) to (7) below.

(1) First, a length (thickness) L1 in the axial direction of the rubber cap is measured.
(2) Next, a spacer is placed on a lower plate of a compression device having an upper plate and the lower plate. A length Ls in the axial direction of the spacer is 75% of L1. That is, Ls = 0.75L1. Moreover, the rubber cap is placed on the lower plate of the compression device such that the axial direction thereof coincides with an up-down direction.
(3) Next, the upper plate of the compression device is moved downward, and the rubber cap and the spacer are interposed and fixed between the upper plate and the lower plate of the compression device while the rubber cap is elastically compressed in the axial direction (the up-down direction) by the upper plate and the lower plate of the compression device. That is, by the compression device, the rubber cap is elastically compressed in the axial direction such that the length in the axial direction of the rubber cap is equal to the length Ls in the axial direction of the spacer (that is, the rubber cap is compressed by 25% in the axial direction), and this state is maintained.
(4) Next, the compression device in which the rubber cap and the spacer have been interposed and fixed as described above is placed in a thermostat bath whose internal temperature is adjusted to 250°C and is left for 67 hours. That is, the state where the rubber cap is compressed by 25% in the axial direction is maintained in a 250°C environment for 67 hours.
(5) After being left in the thermostat bath for 67 hours, the compression device in which the rubber cap and the spacer have been interposed and fixed is taken out from the thermostat bath, and right after that, as the compression of the rubber cap by the compression device is immediately released, the rubber cap is taken out from the compression device and naturally cooled in a normal temperature environment.
(6) A length L2 in the axial direction of the rubber cap is measured when 30 minutes has elapsed after the rubber cap is taken out from the compression device.
(7) Thereafter, the compression set of the rubber cap in the axial direction is calculated using the following arithmetic expression.

$$\text{Compression set (\%)} = \{(L1 - L2)/(L1 - Ls)\} \times 100$$

Moreover, an example of the separator is a tubular separator made of insulating ceramic. In addition, another example of the separator is a separator which includes: a tubular separator body portion obtained by combining a plurality of insulating members made of insulating ceramic; and a body portion holding portion (for example, a body portion holding portion which surrounds the periphery of the separator body portion to hold the tubular shape of the separator body portion) holding a state where the plurality of insulating members are combined into the tubular separator body portion.

Furthermore, the above gas sensor may be a gas sensor that is a NOx sensor for detecting NOx in exhaust gas.

A gas sensor is, in general, exposed at the front side thereof to exhaust gas to detect a specific gas component (oxygen, NOx, or the like) in the exhaust gas. Therefore, the temperature of the front side of the gas sensor becomes high. Meanwhile, in the above-described gas sensor, the separator is interposed and fixed between the rubber cap located on the rear side with respect to the separator and the front side member located on the front side with respect to the separator. In such a gas sensor, the heat of the front side can be transferred to the rubber cap through the front side member and the separator.

In this regard, the above-described gas sensor is a NOx sensor for detecting NOx (for example, the concentration of NOx) in exhaust gas. The NOx sensor is provided downstream of a catalyst in an exhaust pipe, and thus is exposed to relatively low-temperature exhaust gas. Therefore, even if the heat of the front side exposed to the exhaust gas is transferred to the rubber cap through the front side member and the separator, the temperature of the rubber cap is relatively low. Therefore, in the above-described gas sensor which is a NOx sensor, the temperature of the rubber cap is less likely to become high, so that a reduction in the elasticity of the rubber cap due to heat can be decreased, and a state where the separator is fixed can be maintained over a long period of time.

BRIEF DESCRIPTION OF THE DRAWINGS

FIG. 1 is a vertical cross-sectional view of a gas sensor according to an embodiment;
FIG. 2 is a perspective view of a sensor element;
FIG. 3 is a partially-enlarged vertical cross-sectional view of the gas sensor;
FIG. 4 is a diagram illustrating a method for measuring a compression set of a rubber cap;

FIG. 5 is another diagram illustrating the method for measuring the compression set of the rubber cap;

FIG. 6 is still another diagram illustrating the method for measuring the compression set of the rubber cap; and

FIG. 7 is still another diagram illustrating the method for measuring the compression set of the rubber cap.

## DETAILED DESCRIPTION OF THE EXEMPLARY EMBODIMENTS

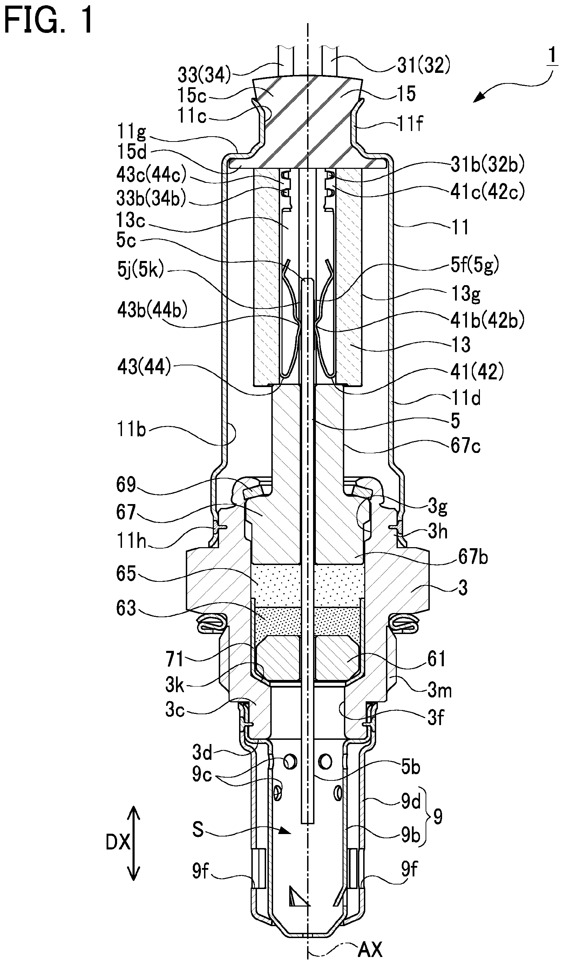

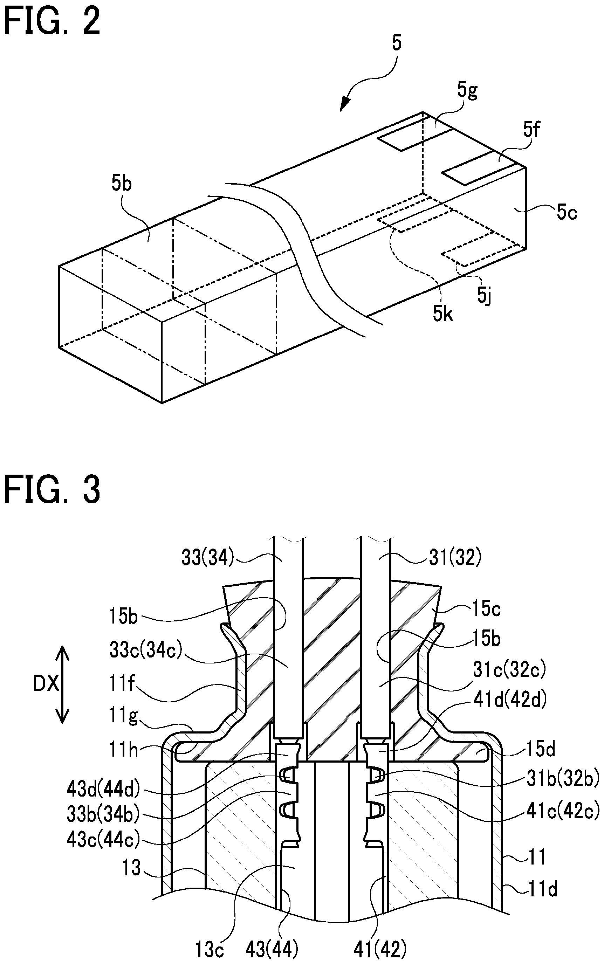

Hereinafter, an embodiment of the present invention will be described with reference to the drawings. FIG. 1 is a vertical cross-sectional view of a gas sensor 1 according to the embodiment, and is a cross-sectional view obtained by cutting the gas sensor 1 in an axial direction DX at a position passing through an axial line AX of the gas sensor 1 (but a sensor element 5 is not cut). In FIG. 1, a lower side is the front side of the gas sensor 1, and the upper side is a rear side of the gas sensor 1. In addition, FIG. 3 is a partially-enlarged vertical cross-sectional view of the gas sensor 1, and is a partially-enlarged cross-sectional view obtained by cutting the gas sensor 1 in the axial direction DX at a position different from that in FIG. 1 (a position that passes through two through holes 15b of a rubber cap 15 and that is a position on the far side of the drawing sheet with respect to FIG. 1, but metal terminals 41 to 44 and lead wires 31 to 34 are not cut).

The gas sensor 1 is mounted on an exhaust pipe of an automobile or the like, which is not shown, such that a portion on the front side thereof is inserted in the exhaust pipe, and is used in a state where the front side thereof is directed downward. Specifically, the gas sensor 1 is a NOx sensor for detecting NOx which is a specific gas component in exhaust gas (gas to be measured). As shown in FIG. 1, the gas sensor 1 includes the sensor element 5, a metal shell 3, a protector 9, and an outer casing 11.

Among these components, the sensor element 5 has a rod shape extending in the axial direction DX (a direction along the axial line AX of the gas sensor 1, the up-down direction in FIG. 1), and has a detection portion 5b covered with a protective layer (not shown) on the front side thereof exposed to a gas to be measured (the exhaust gas). The detection portion 5b detects NOx in the exhaust gas (the gas to be measured). The sensor element 5 further has four electrode terminal portions (a first electrode terminal portion 5f, a second electrode terminal portion 5g, a third electrode terminal portion 5j, a fourth electrode terminal portion 5k) on the rear side thereof (see FIG. 2). The sensor element 5 is inserted in a through hole 3f of the metal shell 3. Specifically, the sensor element 5 is fixed in the through hole 3f of the metal shell 3 in a state where the detection portion 5b located on the front side projects from a front end surface 3d of the metal shell 3 to the front side and the four electrode terminal portions (the first electrode terminal portion 5f to the fourth electrode terminal portion 5k) located on the rear side project from the rear end of the metal shell 3 to the rear side.

The metal shell 3 holds the sensor element 5 (via another member) while surrounding the periphery of the sensor element 5. The metal shell 3 has a tubular shape extending in the axial direction DX, has the through hole 3f extending from the front side to the rear side, and also has a screw portion 3m for fixing the metal shell 3 to an exhaust pipe on the outer surface thereof (see FIG. 1). In the through hole 3f, a shelf portion 3k is formed so as to project radially inward. Furthermore, the metal shell 3 has a cylindrical front side wall portion 3c including the front end surface 3d. The metal shell 3 is formed from a metal material (for example, stainless steel or the like).

Inside the through hole 3f of the metal shell 3, an annular holder 61 made of an insulating material (for example, alumina or the like), talc powder filled layers 63 and 65, and a part (a front side portion 67b) of a tubular sleeve 67 made of an insulating material (for example, alumina or the like) are disposed so as to surround the periphery in the radial direction of the sensor element 5 (see FIG. 1). The sleeve 67 has a cylindrical shape extending in the axial direction DX, and has the annular front side portion 67b and a cylindrical rear side portion 67c having a smaller diameter than the front side portion 67b. A swaging packing 69 is disposed between the front side portion 67b of the sleeve 67 and a rear end portion of the metal shell 3. In addition, a tubular metal holder 71 for holding the talc powder filled layer 63 and the holder 61 is disposed between the holder 61 and the shelf portion 3k of the metal shell 3. The rear end portion of the metal shell 3 is swaged so as to press the front side portion 67b of the sleeve 67 to the front side via the swaging packing 69 (see FIG. 1).

The protector 9 has a tubular shape and is fixed to a portion on the front side of the metal shell 3. Specifically, the protector 9 is fixed to an outer peripheral surface of the front side wall portion 3c of the metal shell 3 by means of welding so as to surround a portion on the front side of the sensor element 5 (a portion projecting from the front end surface 3d of the metal shell 3 to the front side). The protector 9 is formed from a heat resistant material (for example, NCF601 or the like). The protector 9 has a double tubular structure including: a tubular first protector 9b (an inner protector) forming a measurement chamber S in which the detection portion 5b of the sensor element 5 is disposed; and a tubular second protector 9d (an outer protector) surrounding the outer periphery of the first protector 9b. The first protector 9b has a plurality of ventilation holes 9c formed so as to allow the gas to be measured to pass therethrough. The second protector 9d has a plurality of ventilation holes 9f formed so as to allow the gas to be measured to pass therethrough (see FIG. 1).

The outer casing 11 is made of metal and has a cylindrical shape extending in the axial direction DX. The outer casing 11 has a cylindrical body portion 11d, a tubular rear end portion 11f having a smaller diameter than the body portion 11d, and an annular connecting portion 11g connecting the body portion 11d and the rear end portion 11f. The outer casing 11 is mounted (welded) on a portion on the rear side of the metal shell 3 and surrounds a portion on the rear side of the sensor element 5 (see FIG. 1).

The gas sensor 1 further includes four metal terminals (a first metal terminal 41, a second metal terminal 42, a third metal terminal 43, a fourth metal terminal 44), a separator 13, four lead wires (a first lead wire 31, a second lead wire 32, a third lead wire 33, a fourth lead wire 34), and the rubber cap 15 (see FIG. 1). Among these components, the four metal terminals (the first metal terminal 41 to the fourth metal terminal 44) are each made of an elastic heat resistant metal (for example, stainless steel or the like) and have a shape extending in the axial direction DX.

The first metal terminal 41 has a first front side connection portion 41b to which the first electrode terminal portion 5f of the sensor element 5 is connected, and a first rear side connection portion 41c to which the first lead wire 31 (specifically, a metal core wire 31b in the first lead wire 31) is connected (see FIG. 1 and FIG. 3). The second metal terminal 42 has a second front side connection portion 42b to which the second electrode terminal portion 5g of the sensor element 5 is connected, and a second rear side connection portion 42c to which the second lead wire 32 (specifically, a metal core wire 32b in the second lead wire 32) is connected. The third metal terminal 43 has a third front side connection portion 43b to which the third electrode terminal portion 5j of the sensor element 5 is connected, and a third rear side connection portion 43c to which the third lead wire 33 (specifically, a metal core wire 33b in the third lead wire 33) is connected. The fourth metal terminal 44 has a fourth front side connection portion 44b to which the fourth electrode terminal portion 5k of the sensor element 5 is connected, and a fourth rear side connection portion 44c to which the fourth lead wire 34 (specifically, a metal core wire 34b in the fourth lead wire 34) is connected (see FIG. 1 and FIG. 3).

The separator 13 is made of ceramic (for example, alumina, steatite, or the like) and has a cylindrical shape extending in the axial direction DX. The separator 13 is disposed inside the outer casing 11 so as to be spaced apart from an inner peripheral surface 11b of the outer casing 11 (that is, so as not to be in direct contact with the inner peripheral surface 11b of the outer casing 11, see FIG. 1). Moreover, the separator 13 has a through hole 13c penetrating the separator 13 in the axial direction DX. In the through hole 13c, the four front side connection portions (the first front side connection portion 41b to the fourth front side connection portion 44b) of the four metal terminals (the first metal terminal 41 to the fourth metal terminal 44) and a rear end portion 5c having the four electrode terminal portions (the first electrode terminal portion 5f to the fourth electrode terminal portion 5k) of the sensor element 5 are housed (see FIG. 1).

The rubber cap 15 is made of a heat resistant rubber and has a tubular shape (columnar shape) extending in the axial direction DX. The rubber cap 15 has a cylindrical body portion 15c and an annular flange portion 15d located radially outward of the body portion 15c. The rubber cap 15 is fixed to the outer casing 11 so as to close a rear side opening 11c of the outer casing 11 (see FIG. 1). Specifically, the rubber cap 15 is fixed to the outer casing 11 by swaging the rear end portion 11f of the outer casing 11 to the radially inner side. The rubber cap 15 has four through holes 15b penetrating the rubber cap 15 in the axial direction DX (see FIG. 3).

In the gas sensor 1 according to the present embodiment, at least rear end portions (a first rear end portion 41d, a second rear end portion 42d, a third rear end portion 43d, a fourth rear end portion 44d) of the four rear side connection portions (the first rear side connection portion 41c to the fourth rear side connection portion 44c) of the four metal terminals (the first metal terminal 41 to the fourth metal terminal 44) are inserted in the through holes 15b of the rubber cap 15, respectively. In the present embodiment, only the rear end portions (the first rear end portion 41d, the second rear end portion 42d, the third rear end portion 43d, the fourth rear end portion 44d) of the four rear side connection portions (the first rear side connection portion 41c to the fourth rear side connection portion 44c) are inserted in the through holes 15b of the rubber cap 15, respectively (see FIG. 3).

Furthermore, of the four lead wires (the first lead wire 31 to the fourth lead wire 34), portions (a first portion 31c, a second portion 32c, a third portion 33c, a fourth portion 34c) that are located on the rear side with respect to the four rear side connection portions (the first rear side connection portion 41c to the fourth rear side connection portion 44c) and that are inserted in the through holes 15b of the rubber cap 15 are fixed in close contact with the through holes 15b of the rubber cap 15 (tubular surfaces forming the through holes 15b). The four lead wires (the first lead wire 31 to the fourth lead wire 34) extend from the rear side of the through holes 15b of the rubber cap 15 to the outside of the rubber cap 15 (see FIG. 3).

Furthermore, in the gas sensor 1 according to the present embodiment, the separator 13 is interposed and fixed between the rubber cap 15 located on the rear side (an upper side in the FIG. 1) with respect to the separator 13 and elastically compressed in the axial direction DX and the sleeve 67 (a front side member) located on the front side (a lower side in FIG. 1) with respect to the separator 13. That is, the separator 13 is interposed and fixed between the rubber cap 15 and the sleeve 67 (the front side member) so as to be pressed to the front side by the rubber cap 15 elastically compressed in the axial direction DX. As described above, the separator 13 is interposed and fixed between the rubber cap 15 and the sleeve 67 (the front side member) while being pressed to the front side by the elastic reaction force of the rubber cap 15 elastically compressed in the axial direction DX.

Therefore, in the gas sensor 1 according to the present embodiment, the separator 13 is fixed by the rubber cap 15 fixed to the outer casing 11. In other words, the separator 13 is fixed to the outer casing 11 via the rubber cap 15 (see FIG. 1). The outer casing 11 is fixed to the portion on the rear side (an outer casing fixed portion 3h) of the metal shell 3 by means of welding. In addition, the sleeve 67 is also fixed to the metal shell 3 by swaging the rear end portion of the metal shell 3. The metal shell 3 is fixed to an exhaust pipe, which is not shown, by the screw portion 3m. Therefore, the outer casing 11 and the sleeve 67 are fixed to the exhaust pipe through the metal shell 3.

In such a gas sensor 1, when a force is applied from the outside to the gas sensor 1 or vibration is transmitted from the outside to the gas sensor 1, "vibration of the separator 13 relative to the outer casing 11 in a direction (for example, the radial direction of the outer casing 11) crossing the axial direction DX" is less likely to occur. Therefore, it can be said that the gas sensor 1 according to the present embodiment is the gas sensor 1 in which the separator 13 is less likely to vibrate relative to the outer casing 11 in an axial line crossing direction (which is the direction crossing the axial direction DX).

Furthermore, in the four metal terminals (the first metal terminal 41 to the fourth metal terminal 44), the four front side connection portions (the first front side connection portion 41b to the fourth front side connection portion 44b) which are housed inside the separator 13 are also less likely to vibrate relative to the outer casing 11 in the axial line crossing direction (see FIG. 1). In the gas sensor 1, since the rubber cap 15 is fixed to the outer casing 11, the four rear side connection portions (the first rear side connection portion 41c to the fourth rear side connection portion 44c) whose rear end portions (the first rear end portion 41d, the second rear end portion 42d, the third rear end portion 43d, the fourth rear end portion 44d) are inserted in the through holes 15b of the rubber cap 15 are less likely to vibrate relative to the outer casing 11 in the axial line crossing direction (see FIG. 3).

Therefore, in the gas sensor 1 according to the present embodiment, in the four metal terminals (the first metal terminal 41 to the fourth metal terminal 44) extending in the axial direction DX, "vibration of the front side connection portions (the first front side connection portion 41b to the fourth front side connection portion 44b) relative to the rear side connection portions (the first rear side connection portion 41c to the fourth rear side connection portion 44c) in the axial line crossing direction" is less likely to occur, so that stress (for example, shear stress) is less likely to occur in the metal terminals (the first metal terminal 41 to the fourth metal terminal 44), and breakage of the metal terminals (the first metal terminal 41 to the fourth metal terminal 44) is also less likely to occur.

In the gas sensor 1 according to the present embodiment, the rubber cap 15 has the cylindrical body portion 15c and the annular flange portion 15d located radially outward of the body portion 15c. In addition, the outer casing 11 has the cylindrical body portion 11d, the tubular rear end portion 11f having a smaller diameter than the body portion 11d, and the annular connecting portion 11g connecting the body portion 11d and the rear end portion 11f. The flange portion 15d of the rubber cap 15 pressed to the rear side by the separator 13 is in contact with an inner surface 11h (a surface facing the front side) of the connecting portion 11g of the outer casing 11 (see FIG. 1 and FIG. 3). With such a structure, the force received by the rubber cap 15 from the separator 13 (force with which the separator 13 presses the rubber cap 15 to the rear side) can be received by the connecting portion 11g (an inner surface 11h) of the outer casing 11, so that the rubber cap 15 can be prevented from being detached or displaced to the rear side. Accordingly, the separator 13 can be stably interposed and fixed by the rubber cap 15 and the sleeve 67 (the front side member).

Furthermore, in the gas sensor 1 according to the present embodiment, the outer diameter of the separator 13 is made larger than the minimum inner diameter (an inner diameter at the swaged portion) of the rear end portion 11f of the outer casing 11, so that the force received by the rubber cap 15 from the separator 13 (the force with which the separator 13 presses the rubber cap 15 to the rear side) can be more appropriately received by the outer casing 11 (the connecting portion 11g, etc.). Therefore, the separator 13 can be more stably interposed and fixed by the sleeve 67 (the front side member) and the rubber cap 15 elastically compressed in the axial direction DX. The separator 13, the rubber cap 15, and the outer casing 11 are disposed such that the axial lines thereof overlap the axial line AX (coincide with the axial line AX).

Meanwhile, the gas sensor 1 according to the present embodiment is exposed at the portion on the front side thereof to exhaust gas to detect a specific gas component in the exhaust gas. Therefore, the temperature of the portion on the front side of the gas sensor 1 becomes high. In addition, in the gas sensor 1, the separator 13 is interposed and fixed between the rubber cap 15 located on the rear side with respect to the separator 13 and the sleeve 67 located on the front side with respect to the separator 13. In such a gas sensor 1, the heat of the portion on the front side can be transferred to the rubber cap 15 through the sleeve 67 and the separator 13.

In this regard, the gas sensor 1 according to the present embodiment is a NOx sensor for detecting Nox (for example, the concentration of Nox) in exhaust gas. That is, the gas sensor 1 is a Nox sensor for detecting Nox as a specific gas component in exhaust gas. The Nox sensor is provided downstream of a catalyst in an exhaust pipe, and thus is exposed to relatively low-temperature exhaust gas. Therefore, even if the heat of the portion on the front side exposed to the exhaust gas is transferred to the rubber cap 15 through the sleeve 67 and the separator 13, the temperature of the rubber cap 15 is relatively low. Therefore, in the gas sensor 1 which is a Nox sensor, the temperature of the rubber cap 15 is less likely to become high, so that a reduction in the elasticity of the rubber cap 15 due to heat can be decreased, and a state where the separator 13 is fixed can be maintained over a long period of time.

Meanwhile, in the gas sensor 1 according to the present embodiment, as described above, the separator 13 is fixed by using the elastic reaction force of the rubber cap 15 elastically compressed in the axial direction DX. Therefore, in the case where a rubber cap having a large compression set in the axial direction DX is used, the elastic reaction force of the rubber cap may be significantly reduced due to the use of the gas sensor 1, and it may become impossible to properly fix the separator 13.

In this regard, in the gas sensor 1 according to the present embodiment, as a rubber cap, the rubber cap 15 whose compression set (compression set in the axial direction DX) after the rubber cap is compressed by 25% in the axial direction DX for 67 hours in a temperature environment of 250°C is 32.3% or less, is used. Specifically, in the present embodiment, the rubber cap 15 whose compression set measured under the measurement conditions in accordance with JIS K6262 of a measurement temperature of 250°C, a measurement time of 67 hours, and a compression rate of 25%, is 32.3% or less, is used. Accordingly, a reduction in the elastic reaction force of the rubber cap 15 due to the use of the gas sensor 1 (in particular, use in a high temperature environment) can be decreased, so that a state where the separator 13 is fixed can be maintained over a long period of time.

<Measurement of compression set of rubber cap>

Measurement of a compression set was performed for the rubber cap 15 of the present embodiment. Specifically, five rubber caps 15 (samples A to E) were prepared, and the compression sets thereof were measured. The compression sets of the samples A to E were measured under the measurement conditions in accordance with JIS K6262 of a measurement temperature of 250°C, a measurement time of 67 hours, and a compression rate of 25% by a measurement method described in (1) to (7) below.

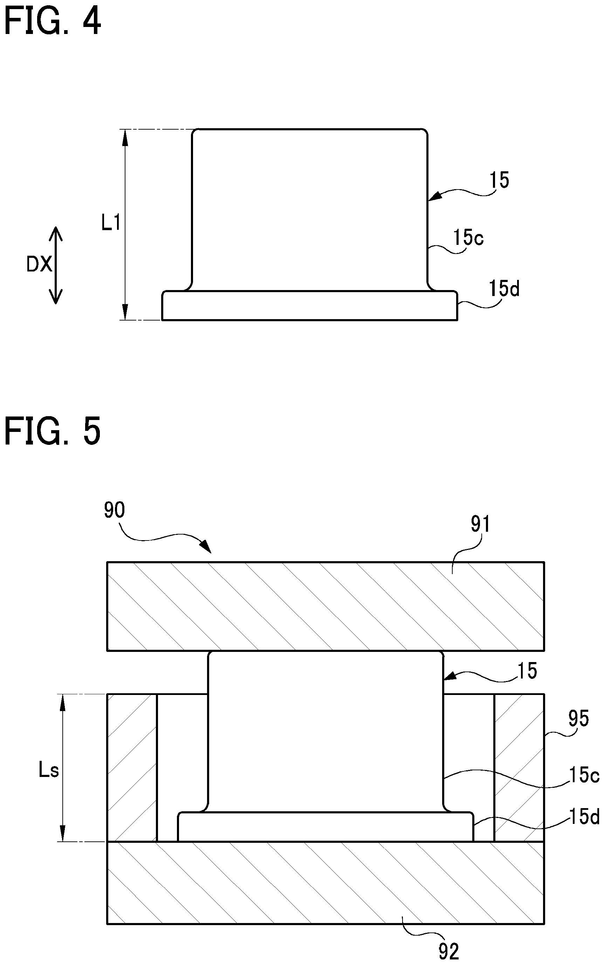

(1) First, as shown in FIG. 4, a length (thickness) L1 in the axial direction DX (an up-down direction in FIG. 4) of each of the rubber caps 15 (samples A to E) is measured.

(2) Next, as shown in FIG. 5, a spacer 95 is placed on a lower plate 92 of a compression device 90 having an upper plate 91 and the lower plate 92. A length (thickness) Ls in the axial direction DX (the up-down direction) of the spacer 95 is 75% of L1. That is, Ls = 0.75L1. Moreover, the rubber cap 15 is placed on the lower plate 92 of the compression device 90 such that the axial direction DX coincides with the up-down direction and the rubber cap 15 is disposed inside the spacer 95.

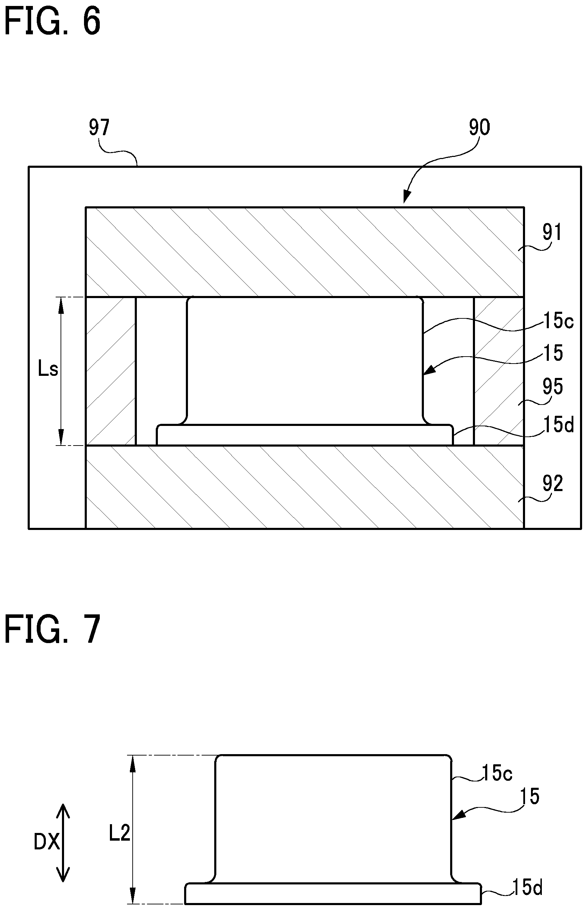

(3) Next, the upper plate 91 of the compression device 90 is moved downward, and the rubber cap 15 and the spacer 95 are interposed and fixed between the upper plate 91 and the lower plate 92 of the compression device 90 while the rubber cap 15 is elastically compressed in the axial direction DX (the up-down direction) by the upper plate 91 and the lower plate 92 of the compression device 90 (see FIG. 6). That is, by the compression device 90, the rubber cap 15 is elastically compressed in the axial direction DX such that the length in the axial direction DX of the rubber cap 15 is equal to the length Ls in the axial direction DX of the spacer 95 (that is, the rubber cap 15 is compressed by 25% in the axial direction DX), and this state is maintained.

(4) Next, the compression device 90 in which the rubber cap 15 and the spacer 95 have been interposed and fixed as described above is placed in a thermostat bath 97 whose internal temperature is adjusted to 250°C, and is left for 67 hours (see FIG. 6). That is, the state where the rubber cap 15 is compressed by 25% in the axial direction DX is maintained in a temperature environment of 250°C for 67 hours.

(5) After being left in the thermostat bath 97 for 67 hours, the compression device 90 in which the rubber cap 15 and the spacer 95 have been interposed and fixed is taken out from the thermostat bath 97, and as the compression of the rubber cap 15 by the compression device 90 is immediately released, the rubber cap 15 is taken out from the compression device 90 and naturally cooled in a normal temperature environment.

(6) Next, as shown in FIG. 7, a length L2 in the axial direction DX of the rubber cap 15 is measured when 30 minutes has elapsed after the rubber cap 15 is taken out from the compression device 90.

(7) Thereafter, the compression set of the rubber cap 15 in the axial direction DX is calculated using the following arithmetic expression.

$$\text{Compression set }(\%) = \{(L1 - L2)/(L1 - Ls)\} \times 100$$

The compression sets (%) of the samples A to E measured as described above are shown in Table 1.

[Table 1]

| Rubber Cap | Permanent Compression Set |
|---|---|
| Sample A | 28.0% |
| Sample B | 32.3% |
| Sample C | 30.0% |
| Sample D | 27.6% |
| Sample E | 29.7% |

As shown in Table 1, the values of the compression sets of the samples A to E were all 32.3% or less. From this result, it can be said that the compression set (the compression set in the axial direction DX) of the rubber cap 15 of the present embodiment after the rubber cap is compressed by 25% in the axial direction DX in a temperature environment of 250°C for 67 hours is 32.3% or less. In the gas sensor 1 in which such a rubber cap 15 is used, a reduction in the elastic reaction force of the rubber cap 15 due to the use of the gas sensor 1 (in particular, use in a high temperature environment) can be decreased, so that a state where the separator 13 is fixed can be maintained over a long period of time.

While the present invention has been described above based on the embodiment, it should be understood that the present invention is not limited thereto but can be applied with modifications appropriately made thereto without departing from the scope of the gist of the present invention. For example, in the embodiment, the tubular separator 13 made of insulating ceramic is exemplified as a separator. However, as the separator, a separator, which includes: a tubular separator body portion obtained by combining a plurality of insulating members made of insulating ceramic; and a body portion holding portion (for example, a body portion holding portion which surrounds the periphery of the separator body portion to hold the tubular shape of the separator body portion) holding a state where the plurality of insulating members are combined into the tubular separator body portion, may be used.

In the embodiment, only the separator 13 is interposed between the rubber cap 15 and the sleeve 67 (the front side member) such that the separator 13 is in direct contact with the rubber cap 15 (the separator 13 is pressed to the front side directly by the rubber cap 15). However, the separator 13 and an other member may be interposed between the rubber cap 15 and a front side member (a member located on the front side with respect to the separator 13) such that the other member is interposed between the separator 13 and the rubber cap 15 and the separator 13 is pressed to the front side indirectly by the rubber cap 15 via the other member.

Reference Signs List

    1 Gas sensor
    3 Metal shell
    5 Sensor element
    5b Detection portion
    5f, 5g, 5j, 5k Electrode terminal portion
    11 Outer casing
    13 Separator
    15 Rubber cap
    31, 32, 33, 34 Lead wire
    41, 42, 43, 44 Metal terminal
    41b, 42b, 43b, 44b Front side connection portion
    41c, 42c, 43c, 44c Rear side connection portion
    67 Sleeve (front side member)
    AX Axial line
    DX Axial direction

**Claims**

**1.** A gas sensor (1) comprising:

a sensor element (5) which extends in an axial direction (DX) and which has an electrode terminal portion (5f, 5g, 5j, 5k) on a rear side of the sensor element;

a metal shell (3) which surrounds a periphery of the sensor element (5);

an outer casing (11) which is made of metal, which has a tubular shape extending in the axial direction (DX), and which is mounted on a portion on the rear side of the metal shell (3);

a metal terminal (41, 42, 43, 44) which extends in the axial direction (DX) and which has a front side connection portion (41b, 42b, 43b, 44b) to which the electrode terminal portion (5f, 5g, 5j, 5k) of the sensor element (5) is connected;

a separator (13) which has a tubular shape extending in the axial direction (DX), which is disposed inside the outer casing (11) so as to be spaced apart from an inner peripheral surface of the outer casing (11), and in which the front side connection portion (41b, 42b, 43b, 44b) of the metal terminal (41, 42, 43, 44) and a portion, of the sensor element (5), having the electrode terminal portion (5f, 5g, 5j, 5k) are housed;

a lead wire (31, 32, 33, 34) which is connected to a rear side connection portion (41c, 42c, 43c, 44c), of the metal terminal (41, 42, 43, 44), located on the rear side of the metal terminal, and which extends to the rear side of the gas sensor; and

a rubber cap (15) which is fixed to the outer casing (11) so as to close a rear side opening of the outer casing (11), **characterized in that**

the separator (13) is interposed and fixed between the rubber cap (15) located on the rear side with respect to the separator (13) and elastically compressed in the axial direction (DX) and a front side member (67) located on a front side with respect to the separator (13), and

a compression set of the rubber cap (15) after the rubber cap (15) is compressed by 25% in the axial direction (DX) in a temperature environment of 250°C for 67 hours is 32.3% or less.

2. The gas sensor (1) according to claim 1, wherein the gas sensor (1) is a NOx sensor for detecting NOx in exhaust gas.

# FIG. 1

1

33(34)
15c
11c
31(32)
15
11f

11g
15d
43c(44c)
33b(34b)
13c
5c
5j(5k)
43b(44b)
43(44)
11b
69
67
11h
65
63

31b(32b)
41c(42c)
11
5f(5g)
13g
41b(42b)
13
41(42)
11d
5
67c
3g
3h
67b
3
67b
3m
3f

71
3k
3c
3d
9c
S
9f

61
3m
3f
5b
9d
9b
9
9f

DX

AX

# FIG. 2

# FIG. 3

# FIG. 4

# FIG. 5

# FIG. 6

# FIG. 7

Europäisches
Patentamt
European
Patent Office
Office européen
des brevets

# EUROPEAN SEARCH REPORT

**Application Number**

EP 22 16 5000

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/288759 A1 (OKUMURA TATSUYA [JP] ET AL) 28 December 2006 (2006-12-28) * abstract; figures 1, 2, 5, 11, 13, 15, 17 * * paragraphs [0065], [0073] – [0075], [0087], [0093], [0101] * | 1,2 | INV. G01N27/407 ADD. G01N33/00 |
| A | US 2016/013460 A1 (FUKUNAGA ATSUSHI [JP] ET AL) 14 January 2016 (2016-01-14) * abstract; figure 1 * * paragraphs [0034] – [0036] * | 1,2 | |
| X | JP 2010 266361 A (NGK SPARK PLUG CO) 25 November 2010 (2010-11-25) * abstract; figures 1, 3 * * paragraphs [0016] – [0021], [0024], [0032], [0033] * | 1,2 | |
| X | DE 11 2019 002895 T5 (NGK SPARK PLUG CO [JP]) 18 February 2021 (2021-02-18) * abstract; figure 1 * * paragraphs [0029] – [0039] * | 1,2 | TECHNICAL FIELDS SEARCHED (IPC) G01N |
| A,D | JP 2019 002936 A (NGK INSULATORS LTD) 10 January 2019 (2019-01-10) * figure 1 * | 1,2 | |

The present search report has been drawn up for all claims

| Place of search | Date of completion of the search | Examiner |
|---|---|---|
| Munich | 8 September 2022 | Hanisch, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

......................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 5000

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2022

| Patent document cited in search report | | Publication date | Patent family member(s) | | Publication date |
|---|---|---|---|---|---|
| US 2006288759 | A1 | 28-12-2006 | CN | 2932386 Y | 08-08-2007 |
| | | | EP | 1729120 A1 | 06-12-2006 |
| | | | JP | 4494403 B2 | 30-06-2010 |
| | | | JP | WO2005090960 A1 | 07-02-2008 |
| | | | US | 2006288759 A1 | 28-12-2006 |
| | | | WO | 2005090960 A1 | 29-09-2005 |
| US 2016013460 | A1 | 14-01-2016 | CN | 105103338 A | 25-11-2015 |
| | | | JP | 2014170648 A | 18-09-2014 |
| | | | KR | 20150123793 A | 04-11-2015 |
| | | | US | 2016013460 A1 | 14-01-2016 |
| | | | WO | 2014132684 A1 | 04-09-2014 |
| JP 2010266361 | A | 25-11-2010 | NONE | | |
| DE 112019002895 | T5 | 18-02-2021 | CN | 112272769 A | 26-01-2021 |
| | | | DE | 112019002895 T5 | 18-02-2021 |
| | | | JP | 6971203 B2 | 24-11-2021 |
| | | | JP | 2019211413 A | 12-12-2019 |
| | | | US | 2021247339 A1 | 12-08-2021 |
| | | | WO | 2019234972 A1 | 12-12-2019 |
| JP 2019002936 | A | 10-01-2019 | JP | 6674508 B2 | 01-04-2020 |
| | | | JP | 2019002936 A | 10-01-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019002936 A